(19) Europäisches Patentamt
European Patent Office
Office européen des brevets

(11) Veröffentlichungsnummer: **0 047 337**
**A1**

# (12) EUROPÄISCHE PATENTANMELDUNG

(21) Anmeldenummer: **80106382.7**

(51) Int. Cl.³: **C 07 C 87/60**

(22) Anmeldetag: **20.10.80**

(30) Priorität: **09.09.80 DE 3033862**

(43) Veröffentlichungstag der Anmeldung: **17.03.82**
**Patentblatt 82/11**

(84) Benannte Vertragsstaaten: **BE CH DE FR GB LI SE**

(71) Anmelder: **BAYER AG, Zentralbereich Patente, Marken und Lizenzen, D-5090 Leverkusen 1, Bayerwerk (DE)**

(72) Erfinder: **Goldschmitt, Ernst, Dr., Andreas-Gryphius-Strasse 9, D-5000 Köln 80 (DE)**
Erfinder: **Schössler, Willi, Dr., Hahnenweg 2, D-5000 Köln 80 (DE)**
Erfinder: **Blank, Heinz-Ulrich, Dr., Am Geusfelde 35, D-5068 Odenthal (DE)**

(54) **Verfahren zur Herstellung von 3-Nitro-4-aminotoluol.**

(57) Die vorliegende Erfindung betrifft ein Verfahren zur Herstellung von 3-Nitro-4-aminotoluol aus 4-Tolylcarbaminsäureestern durch Nitrierung in Gegenwart von inerten, mit Wasser nicht mischbaren organischen Lösungsmitteln.

EP 0 047 337 A1

0047337



BAYER AKTIENGESELLSCHAFT 5090 Leverkusen, Bayerwerk
Zentralbereich
Patente, Marken und Lizenzen
Bg/bc/c 8.09.80

## Verfahren zur Herstellung von 3-Nitro-4-aminotoluol

Die vorliegende Erfindung betrifft ein Verfahren zur Herstellung von 3-Nitro-4-aminotoluol aus 4-Tolylcarbaminsäureestern durch Nitrierung in Gegenwart von inerten, mit Wasser nicht mischbaren organischen Lösungsmitteln.

Es ist bekannt, 3-Nitro-4-aminotoluol durch Nitrieren von 4-Acetylaminotoluol mit Hilfe eines Säuregemisches aus Salpetersäure und Schwefelsäure in einem inerten Lösungsmittel, wie Methylenchlorid, herzustellen (DE-AS 2 226 405).

Dieses Verfahren hat jedoch den Nachteil, daß neben dem gewünschten 3-Nitro-4-acetylaminotoluol noch das 2-Nitro-4-acetylaminotoluol als Nebenprodukt entsteht, welches nach der alkalischen Entacetylierung die Qualität des 3-Nitro-4-aminotoluols beeinträchtigt.

Weiterhin sind die Ausbeuten an gewünschtem Reaktionsprodukt mit 88 bis 91 % der Theorie für ein wirtschaftliches Verfahren nicht befriedigend.

Ferner ist bekannt, 3-Nitro-4-acetylaminotoluol herzustellen, indem in einem Eintopfverfahren, ausgehend von p-Aminotoluol, dieses zuerst mit Acetanhydrid in Gegenwart von Monochlorbenzol bei erhöhter Temperatur acetyliert und anschließend mit 80 %iger Salpetersäure bei 50°C nitriert wird (vgl. US-PS 2 459 002, Beispiel 4).

Nachteilig bei diesem Verfahren sind die geringen Ausbeuten und die schlechten Qualitäten an 3-Nitro-4-aminotoluol, wie eine Nacharbeitung des Beispiels 4 der US-PS zeigt (in Beispiel 4 der US-PS fehlt nämlich eine Angabe zur Ausbeute und Produktqualität).

Da bei dem Verfahren der US-PS 2 459 002 die Acylierung und Nitrierung nacheinander, d.h. ohne Isolierung des acylierten Zwischenprodukts durchgeführt werden, besteht das Nitriergemisch immer aus überschüssigem Acylierungsmittel, freigesetzter organischer oder anorganischer Säure und Salpetersäure. Dies hat jedoch den Nachteil, daß neben den geringen Ausbeuten und der schlechten Qualitäten an 3-Nitro-4-aminotoluol bei der anschließenden Neutralisation des Reaktionsgemisches mehr Alkali benötigt wird als eigentlich für die Neutralisation der Salpetersäure erforderlich ist. Durch die Neutralisation geht das überschüssige Acylierungsmittel und die freigesetzte organische oder anorganische Säure für eine weitere wirtschaftliche Nutzung verloren und zusätzlich wird das Abwasser durch die bei der Neutralisation gebildeten Salze belastet.

Weiterhin wurde bei der Nacharbeitung des Beispiels 4 der US-PS beobachtet, daß, wenn das 3-Nitro-4-acetylaminotoluol ohne Zwischenisolierung in saurem Medium gespalten wird, zum Teil Verharzung eintritt. Zur Isolierung des 3-Nitro-4-aminotoluols ist es daher erforderlich, die Reaktionslösung zuvor mit Aktivkohle zu behandeln.

Es wurde nun ein Verfahren zur Herstellung von 3-Nitro-4-aminotoluol durch Nitrieren von 4-Tolylcarbaminsäureestern und anschließender alkalischer Verseifung und Aufarbeitung gefunden, das dadurch gekennzeichnet ist, daß man die Nitrierung in Gegenwart von inerten, mit Wasser nicht mischbaren organischen Lösungs- und/oder Verdünnungsmitteln mit 50 bis 100 gew.-%iger Salpetersäure bei Temperaturen im Bereich von -10 bis +45°C durchführt.

Als 4-Tolylcarbaminsäureester können solche der allgemeinen Formel

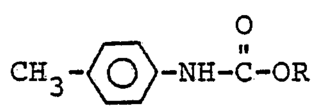

,

worin

R für einen cycloaliphatischen oder aliphatischen Rest mit 1 bis 8 Kohlenstoffatomen, bevorzugt 1 bis 6 Kohlenstoffatomen, steht,

in das erfindungsgemäße Verfahren eingesetzt werden.

Als cycloaliphatische Reste seien genannt: Cyclopentyl, Cyclohexyl, Cycloheptyl, 1-, 2- und 3-Methylcyclopentyl und 1- und 2-Methylcyclohexyl, bevorzugt Cyclohexyl;

als aliphatische Reste seien genannt: Methyl, Ethyl, n-Propyl, iso-Propyl, n-Butyl, iso-Butyl, tert.-Butyl, n-Pentyl, iso-Pentyl, n-Hexyl, iso-Hexyl und 2.2-Dimethyl-butyl-(3), bevorzugt Methyl und Ethyl.

Bevorzugt werden solche 4-Tolylcarbaminsäureester in das erfindungsgemäße Verfahren eingesetzt, deren aliphatische oder cycloaliphatische Reste nach der Spaltung leicht aus dem Reaktionsgemisch durch Destillation, Phasentrennung und/oder durch Extraktion zu entfernen sind.

Zum Beispiel können folgende Tolylcarbaminsäureester eingesetzt werden: 4-Tolylcarbaminsäuremethyl-, -ethyl-, -n-propyl-, -iso-propyl-, -tert.-butyl-, -n-butyl-, -iso-butyl-, -n-pentyl-, -iso-pentyl-, -n-hexyl- und -cyclohexylester. Besonders bevorzugt wird der Methyl- und Ethylester der 4-Tolylcarbaminsäure eingesetzt.

Die Salpetersäure wird in das erfindungsgemäße Verfahren bevorzugt in einer Konzentration von 65 bis 100 Gew.-% eingesetzt, wobei die Nitrierung mit 98 %iger Salpetersäure besonders bevorzugt ist.

Nach dem erfindungsgemäßen Verfahren werden pro Mol 4-Tolylcarbaminsäureester mindestens 1,5 Mol 100 gew.-%ige Salpetersäure, bevorzugt 1,5 bis 5 Mol, besonders bevorzugt 1,6 bis 3,5 Mol, 100 gew.-%iger Salpetersäure eingesetzt.

Bei Verwendung von 50 gew.-%iger Salpetersäure werden mindestens 3 Mol Salpetersäure pro Mol 4-Tolylcarbaminsäureester eingesetzt. Bevorzugt werden pro Mol 4-Tolylcarbaminsäureester 3 bis 10 Mol, besonders bevorzugt 3,5 bis 7 Mol, 50 gew.-%ige Salpetersäure eingesetzt. Für Salpetersäurekonzentrationen, die zwischen 50 und 100 Gew.-% liegen, wird zweckmäßigerweise das günstigste Molverhältnis von Salpetersäure zu 4-Tolylcarbaminsäureester durch Vorversuche ermittelt.

Beispielsweise beträgt das Molverhältnis von 98 gew.-%iger Salpetersäure zu 4-Tolylcarbaminsäureester 1,6 bis 5:1, bevorzugt 1,7 bis 4:1.

Als inerte, mit Wasser nicht mischbare organische Lösungs- und/oder Verdünnungsmittel werden bevorzugt halogenierte aliphatische Kohlenwasserstoffe mit 1 bis 6, bevorzugt 1 bis 3, Kohlenstoffatomen in das erfindungsgemäße Verfahren eingesetzt. Die Kohlenwasserstoffe können durch Fluor, Chlor, Brom, bevorzugt durch Fluor, Chlor, ein- oder mehrfach substituiert sein. Zum Beispiel seien folgende halogenierte aliphatische Kohlenwasserstoffe genannt: Chloroform, 1,1-Dichlorethan, 1,2-Dichlorethan, 1,1,1-Trichlorethan, Methylenchlorid, 1,1-Dichlorpropan, 2,2-Dichlorpropan und Monofluortrichlormethan.

Besonders bevorzugt wird Methylenchlorid in das erfindungsgemäße Verfahren eingesetzt.

Die inerten organischen Lösungs- und/oder Verdünnungsmittel können sowohl einzeln als auch im Gemisch untereinander eingesetzt werden.

Die Menge der inerten organischen Lösungs- und/oder Verdünnungsmittel ist nicht kritisch und kann in weiten Bereichen variieren. Im allgemeinen beträgt die Menge an inerten organischen Lösungs- und/oder Verdünnungsmitteln etwa 400 bis etwa 2000 ml pro Mol 4-Tolylcarbaminsäureester. Bevorzugt werden auf 1 Mol 4-Tolylcarbaminsäureester 450 bis 1000 ml, besonders bevorzugt 500 bis 800 ml inerte organische Lösungs- und/oder Verdünnungsmittel eingesetzt.

Die erfindungsgemäße Nitrierung wird im allgemeinen bei Temperaturen von etwa -10 bis etwa +45°C, bevorzugt bei 0 bis 40°C, besonders bevorzugt bei 10 bis 25°C, durchgeführt.

Die Reaktionszeit der Nitrierung hängt im wesentlichen von der Salpetersäurekonzentration und der Menge der eingesetzten Salpetersäure ab. Die Reaktionszeit ist um so kürzer, je höher konzentriert die Salpetersäure und je größer der Überschuß an Salpetersäure bezogen auf das Ausgangsprodukt ist. Im allgemeinen betragen die Reaktionszeiten etwa 15 Minuten bis etwa 3 Stunden.

Das erfindungsgemäße Verfahren kann im angegebenen Temperaturbereich sowohl bei Normaldruck als auch bei vermindertem oder erhöhtem Druck, bespielsweise bei Drücken im Bereich von etwa 0,02 bis 1,2 bar, bevorzugt bei 0,05 bis 1 bar, durchgeführt werden.

Neben der diskontinuierlichen Fahrweise ist auch eine kontinuierliche Durchführung des erfindungsgemäßen Verfahrens möglich.

Das erfindungsgemäße Verfahren kann in der Weise durchgeführt werden, daß man das Einsatzprodukt gelöst oder suspendiert in dem inerten organischen Lösungs- und/oder Verdünnungsmittel vorlegt und mit Salpetersäure im zuvor beschriebenen Temperaturbereich versetzt. Dabei ist es im allgemeinen nicht erforderlich, das Reaktionsgemisch zu kühlen, da die Reaktionswärme durch das siedende inerte organische Lösungs- und/oder Verdünnungsmittel abgeführt werden kann.

Bei Verwendung von höherkonzentrierter Salpetersäure kann es vorteilhaft sein, wenn man zu einer Suspension bzw. Lösung von Ausgangsprodukt und inertem organischem Lösungs- und/oder Verdünnungsmittel eine Mischung von Salpetersäure und inertem organischem Lösungs- und/oder Verdünnungsmittel zudosiert. In diesem Fall kann die Qualität an 3-Nitro-4-aminotoluol noch verbessert werden.

Es ist aber auch möglich, die Salpetersäure vorzulegen, gegebenenfalls im Gemisch mit einem inerten organischen Lösungs- und/oder Verdünnungsmittel, und das Einsatzprodukt zusammen mit dem inerten organischen Lösungs- und/oder Verdünnungsmittel als Lösung oder Suspension zuzugeben.

Bei der Durchführung der Nitrierung ist es günstig,

wenn sich die Konzentration an Salpetersäure während der Reaktion so wenig wie möglich verändert. Man arbeitet daher bei Verwendung einer weniger als 98 bis 100 gew.-%igen Salpetersäure z.B. so, daß man in die vorgelegte Suspension oder Lösung von 4-Tolylcarbaminsäureester und einem inerten organischen Lösungs- und/ oder Verdünnungsmittel pro Mol 4-Tolyl-carbaminsäureester zunächst die um 1 Mol verringerte Salpetersäuremenge der betreffenden Konzentration und anschließend während der Reaktion noch 1 Mol 98 bis 100 gew.-%iger Salpetersäure zudosiert.

Eine bevorzugte Verfahrensvariante besteht darin, daß man den 4-Tolylcarbaminsäureester und die Salpetersäure synchron in vorgelegtes inertes organisches Lösungs- und/oder Verdünnungsmittel, das gegebenenfalls einen Teil der zur Nitrierung erforderlichen Salpetersäure und gegebenenfalls noch Zusätze wie salpetrige Säure und/oder deren Salze und/oder deren Anhydrid und/oder Nitrosylschwefelsäure enthält, eindosiert.

Der 4-Tolylcarbaminsäureester kann dabei gelöst oder suspendiert in einem inerten organischen Lösungs- und/ oder Verdünnungsmittel eingesetzt werden.

Es ist selbstverständlich auch möglich, den 4-Tolylcarbaminsäureester in fester Form oder als Schmelze in das erfindungsgemäße Verfahren einzusetzen.

Die Menge der mit dem inerten organischen Lösungs- und/ oder Verdünnungsmittel vorgelegten Salpetersäure kann

in weiten Bereichen schwanken. Im allgemeinen beträgt die Menge an vorgelegter Salpetersäure etwa 1 bis 70 %, bevorzugt 2 bis 50 %, besonders bevorzugt 5 bis 40 %, der zur Nitrierung erforderlichen Gesamtmenge.

Die Menge der oben erwähnten Zusätze, die auch der einzudosierenden Salpetersäure zugegeben werden können, beträgt im allgemeinen 0,01 bis 5 Mol-%, bevorzugt 0,03 bis 1 Mol-%, besonders bevorzugt 0,05 bis 0,5 Mol-%, bezogen auf eingesetztes Ausgangsprodukt.

Die Aufarbeitung des Nitriergemisches kann in der Weise erfolgen, daß das Reaktionsgemisch in eine wäßrige, etwa 5 bis 25 gew.-%ige (die Angaben in Gew.-% beziehen sich auf den Gehalt an freier Base nach Neutralisation der überschüssigen Salpetersäure), bevorzugt 10 bis 20 gew.-%ige (s.o.) Lösung oder Suspension eines Ammonium-, Alkali- und/oder Erdalkalihydroxids und/oder Carbonats, bevorzugt in eine wäßrige Alkalihydroxidlösung, besonders bevorzugt in eine wäßrige Natronlauge, gegeben wird.

Dabei wird die Temperatur vorzugsweise so gewählt (z.B. 50 bis 95°C), daß das inerte organische Lösungs- und/oder Verdünnungsmittel zusammen mit Wasser abdestilliert.

Die anschließende alkalische Abspaltung der Schutzgruppe wird bei etwa 80 bis 100°C, vorzugsweise bei 90 bis 95°C, durchgeführt. Danach wird bei etwa 0 bis 80°C, bevorzugt bei 50 bis 70°C, das ausgefallene Reaktionsprodukt abfiltriert und mit Wasser alkalifrei gewaschen.

Statt die alkalische Abspaltung der Schutzgruppe in einem wäßrigen System vorzunehmen, kann es vorteilhaft sein, diese in einem wäßrig/alkoholischen oder im alkoholischen System durchzuführen.

Als geeignete Alkohole kommen hierfür vorzugsweise aliphatische Alkohole mit 1 bis 4 Kohlenstoffatomen in Betracht, welche bei oder nach erfolgter Abspaltung der Schutzgruppe zusammen mit Wasser abdestilliert werden können.

Bevorzugt wird für die Spaltung Methanol, Ethanol, n-Propanol, iso-Propanol und tert.-Butanol, besonders bevorzugt Methanol, eingesetzt.

Die Aufarbeitung des Nitriergemisches kann auch derart durchgeführt werden, daß das gesamte Reaktionsgemisch mit Wasser nahezu säurefrei gewaschen wird. Bevorzugt wird die überschüssige Salpetersäure und die in geringen Mengen enthaltenen Diazoniumsalze mit möglichst wenig Wasser diskontinuierlich oder kontinuierlich extrahiert. Auf diese Weise erhält man eine konzentrierte wäßrige Salpetersäurelösung, aus der Salpetersäure von etwa 55 bis etwa 68 Gew.-% sehr wirtschaftlich zurückgewonnen werden kann.

Das mit Wasser extrahierte Nitriergemisch kann, wie zuvor beschrieben, in einem wäßrigen System aufgearbeitet werden.

Bei der Aufarbeitung des Nitriergemisches kann es vorteilhaft sein, in dem Reaktionsgemisch zunächst die überschüssige Salpetersäure mit Ammoniak oder einer wäßrigen Lösung eines Ammonium- oder eines Alkalihydroxids, bevorzugt mit einer wäßrigen Alkalihydroxidlösung, besonders bevorzugt mit wäßrigem Natriumhydroxid, teilweise oder vollständig zu entfernen.

Das teilweise oder vollständig neutralisierte Nitriergemisch kann evtl. nach Versetzen mit Wasser, wie zuvor beschrieben, in einem wäßrigen System aufgearbeitet werden.

Es ist natürlich auch möglich, den 2-Nitro-4-tolylcarbaminsäureester aus dem Nitriergemisch, nachdem dieses durch die zuvor beschriebene Extraktion oder Neutralisation vollständig von der überschüssigen Salpetersäure befreit wurde, durch Abdestillieren des inerten organischen Lösungs- und/oder Verdünnungsmittels zu isolieren.

Dieses isolierte Festprodukt kann, wie zuvor beschrieben, in einem wäßrigen, wäßrig/alkoholischen oder alkoholischen System aufgearbeitet werden.

Weiterhin kann es vorteilhaft sein, während der alkalischen Spaltung eine Wasserdampfdestillation bei Normaldruck oder vermindertem Druck durchzuführen.

Nach erfolgter alkalischer Spaltung kann es auch vorteilhaft sein, das gespaltene Produkt beispielsweise durch eine Flüssigphasentrennung, bevorzugt unter Druck, abzutrennen, oder in einem geeigneten, mit Wasser nicht mischbaren organischen Lösungsmittel, wie Methylenchlorid, Chlorbenzol, Dichlorbenzol oder Toluol entweder bei Raumtemperatur oder erhöhter Temperatur (30 bis 130°C) aufzunehmen, die organische Phase abzutrennen, mit Wasser alkalifrei zu waschen und das 3-Nitro-4-aminotoluol anschließend z.B. durch Kristallisation oder durch Destillation, zu isolieren.

Die Ausbeute an 3-Nitro-4-aminotoluol nach dem erfindungsgemäßen Verfahren beträgt bis zu 95 % der Theorie und es werden Reinheiten bis zu 99,9 % erreicht.

Falls es erforderlich sein sollte, kann das erhaltene 3-Nitro-4-aminotoluol noch weiter durch Umkristallisation, Sublimation oder Destillation gereinigt werden.

Gegenüber den Verfahren nach dem Stand der Technik bietet das erfindungsgemäße Verfahren wesentlich Vorteile. So wird beim erfindungsgemäßen Verfahren eine größere Ausbeute und eine höhere Produktqualität (Reinheit) erzielt. Weiterhin wird bei dem erfindungsgemäßen Verfahren auf die Verwendung von Mineralsäuren als Lösungs- oder Verdünnungsmittel vollständig verzichtet. Dies bedingt gegenüber dem Stand der Technik nicht nur eine kostenmindernde Einsparung an Mineralsäure, sondern löst gleichzeitig die Probleme, die sonst mit der Aufarbeitung oder Beseitigung derartiger Säuren verbunden sind.

Bei dem erfindungsgemäßen Verfahren ist es überraschend, daß der 4-Tolylcarbaminsäureester in einem inerten organischen Lösungs- und/oder Verdünnungsmittel auch bei Verwendung von verdünnter Salpetersäure unter milden Reaktionsbedingungen vollständig nitriert wird.

Ein besonderer Vorteil des erfindungsgemäßen Verfahrens besteht darin, daß ausgehend von 4-Tolylcarbaminsäureestern gegenüber den Verfahren nach dem Stand der Technik nach der alkalischen Schutzgruppenabspaltung das Abwasser wesentlich weniger organisches Material enthält, da der gebildete Alkohol leicht zurückgewonnen werden kann und das gleichzeitig gebildete Kohlendioxid von der Natronlauge in das Carbonat übergeführt wird, und somit keinen BSB (biologischer Sauerstoffbedarf)-Verbrauch erfordert.

Außerdem ist es außerordentlich überraschend, daß bei dem erfindungsgemäßen Verfahren keine Ablagerungen von festen Diazoniumsalzen beobachtet werden konnten. Dies würde nämlich, wie in der DE-AS 2 226 405 ausdrücklich erwähnt, wegen der Gefährlichkeit der festen Diazoniumsalze, eine technische Durchführung des Verfahrens ausschließen.

3-Nitro-4-aminotoluol ist ein wichtiges Ausgangsprodukt zur Herstellung von Farbstoffen (s. Ullmanns Enzyklopädie der technischen Chemie, 3. Auflage, Band 12, Seite 767).

Die nachfolgenden Beispiele sollen das erfindungsgemäße Verfahren erläutern, ohne es jedoch auf diese Beispiele einzuschränken.

Beispiel 1

In einem 2 l Vierhalskolben, ausgerüstet mit Rührer, Rückflußkühler, Thermometer und zwei Tropftrichtern werden 100 ml Methylenchlorid vorgelegt und bei Normaldruck innerhalb von 1 Stunde werden 165,2 g (1 Mol) 4-Tolylcarbaminsäuremethylester gelöst in 500 ml Methylenchlorid und 102,9 g 98 %iger (1,6 Mol) Salpetersäure gelöst in 200 ml Methylenchlorid simultan bei 10°C zugetropft, wobei die Temperatur durch externe Kühlung gehalten wird. Nach beendetem Eintropfen läßt man das Nitriergemisch auf 20°C kommen, rührt noch 1,75 Stunden nach und die erhaltene Lösung läßt man unter Rühren bei 50 bis 60°C in verdünnte Natronlauge, bestehend aus 480 ml Wasser und 144 g (3,6 Mol) NaOH einfließen, wobei Methylenchlorid mit wenig Wasser abdestilliert. Nachdem die gesamte Menge an Methylenchlorid abdestilliert ist, wird die Temperatur bis auf 95°C gesteigert und 3 Stunden gehalten, wobei gleichzeitig das bei der Hydrolyse des 2-Nitro-4-tolylcarbaminsäuremethylesters zur Aminoverbindung gebildete Methanol abdestilliert wird. Es wird auf 60°C abgekühlt, 30 Minuten bei 60°C nachgerührt, abgesaugt und mit Wasser alkalifrei gewaschen. Das Feuchtprodukt wird bis zur Gewichtskonstanz im Vakuum bei 60°C getrocknet. Ausbeute: Produkt trocken 145,4 g (94,9 % der Theorie); Reinheit: 99,3 %.

Beispiel 2

In einem 2 l Vierhalskolben, ausgerüstet mit Rührer, Rückflußkühler, Thermometer und zwei Tropftrichtern werden 100 ml Methylenchlorid vorgelegt und bei Normaldruck innerhalb von 1 Stunde werden 165,2 g (1 Mol) 4-Tolylcarbaminsäuremethylester gelöst in 400 ml Methylenchlorid und 109,3 g 98 %ige (1,7 Mol) Salpetersäure gelöst in 150 ml Methylenchlorid simultan bei 20°C zugetropft, wobei die Temperatur durch externe Kühlung gehalten wird. Nach beendetem Eintropfen wird noch 1 Stunde bei 20°C nachgerührt, die erhaltene Lösung zweimal mit je 100 ml Wasser extrahiert und anschließend das organische Lösungsmittel abdestilliert. Ausbeute: 209,3 g Rohprodukt vom Schmp. 96 bis 102°C. Dieses Rohprodukt wird in verdünnte Natronlauge, bestehend aus 450 ml Wasser und 112 g (2,8 Mol) NaOH eingetragen, auf 95°C erhitzt und diese Temperatur 3 Stunden gehalten, wobei das bei der Hydrolyse des 2-Nitro-4-tolylcarbaminsäuremethylesters zur Aminoverbindung gebildete Methanol abdestilliert wird. Es wird auf 60°C abgekühlt, 30 Minuten bei 60°C nachgerührt, abgesaugt und mit Wasser alkalifrei gewaschen. Das Feuchtprodukt wird bis zur Gewichtskonstanz im Vakuum bei 60°C getrocknet.
Ausbeute: Produkt trocken 144,4 g (94,6 % der Theorie); Reinheit: 99,7 %.

Beispiel 3

Es wurde wie in Beispiel 2 verfahren, jedoch wurde das nach zweimaliger Extraktion mit je 100 ml Wasser erhaltene Nitriergemisch ohne Zwischenisolierung unter Rühren bei 50 bis 60°C in verdünnte Natronlauge, bestehend aus 480 ml Wasser und 120 g (3,0 Mol) NaOH einfließen gelassen und weiter wie im Beispiel 1 beschrieben aufgearbeitet.
Ausbeute: Produkt trocken 144,7 g (94,9 % der Theorie); Reinheit: 99,8 %.

Beispiel 4

In einem 2 l Vierhalskolben ausgerüstet mit Rührer, Rückflußkühler, Thermometer und zwei Tropftrichtern werden 200 ml.Methylenchlorid, 12,2 g 98 %ige (0,19 Mol) Salpetersäure und 0,17 g 40,83 %ige (0,001 Mol) wäßrige Natriumnitritlösung vorgelegt, bei vermindertem Druck (400 mbar) zum Sieden erhitzt (18°C) und innerhalb von 40 Minuten werden 165,2 g (1 Mol) 4-Tolylcarbaminsäuremethylester gelöst in 400 ml Methylenchlorid und 110,0 g 98 %ige (1,71 Mol) Salpetersäure, welche durch den Rückflußkühler eindosiert wird, simultan bei 18°C zugetropft. Nach beendetem Eintropfen wird noch 1 Stunde bei gleicher Temperatur und gleichem Druck nachgerührt, die erhaltene Lösung zweimal mit je 100 ml Wasser extrahiert und die organische Lösung läßt man unter Rühren bei 50 bis 60°C in verdünnte Natronlauge, bestehend aus 480 ml Wasser und 120 g (3,0 Mol) NaOH einfließen,

wobei Methylenchlorid mit wenig Wasser abdestilliert. Anschließend wird weiter wie in Beispiel 1 beschrieben aufgearbeitet.
Ausbeute: Produkt trocken 144,8 g (95,1 % der Theorie); Reinheit: 99,9 %.

Beispiel 5

In einem 2 l Vierhalskolben ausgerüstet mit Rührer, Rückflußkühler, Thermometer und zwei Tropftrichtern werden 100 ml Methylenchlorid vorgelegt und bei Normaldruck innerhalb von 1 Stunde werden 179,2 g (1 Mol) 4-Tolylcarbaminsäureethylester gelöst in 400 ml Methylenchlorid und 109,3 g 98 %ige (1,7 Mol) Salpetersäure gelöst in 150 ml Methylenchlorid simultan bei 20°C zugetropft, wobei die Temperatur durch externe Kühlung gehalten wird. Nach beendetem Eintropfen wird noch 1 Stunde bei 20°C nachgerührt, die erhaltene Lösung zweimal mit je 100 ml Wasser extrahiert und anschließend das organische Lösungsmittel abdestilliert.
Ausbeute: 228 g Rohprodukt vom Schmp. 56 bis 58°C.

Die Lösung dieses Rohproduktes in 400 ml Methylenchlorid wird in verdünnte Natronlauge bestehend aus 550 ml Methanol und 100 g (2,5 Mol) NaOH bei ca. 40°C einlaufen gelassen und danach noch 30 Minuten bei Rückflußtemperatur (42 bis 48°C) nachgerührt, wodurch sich der Kolbeninhalt dunkelrot färbt und Natriumcarbonat ausfällt. Anschließend wird das Methylenchlorid bei Normaldruck abdestilliert und die Suspension mit 1 l Wasser versetzt, wodurch das Natriumcarbonat in Lösung geht.

Es wird auf 20°C abgekühlt, 30 Minuten bei 20°C nachgerührt, abgesaugt und mit Wasser alkalifrei gewaschen. Das Feuchtprodukt wird bis zur Gewichtskonstanz im Vakuum bei 60°C getrocknet.
Ausbeute: Produkt trocken 142,0 g (92,6 % der Theorie); Reinheit: 99,2 %.

Beispiel 6

Es wurde wie in Beispiel 5 verfahren, jedoch wurde die zweimal mit je 100 ml Wasser extrahierte Methylenchloridlösung ohne Zwischenisolierung direkt in vorgelegte verdünnte Natronlauge bestehend aus 550 ml Methanol und 100 g (2,5 Mol) NaOH bei ca. 40°C einlaufen gelassen.

Die Ausbeute und Reinheit des getrockneten Produktes waren praktisch die gleichen wie bei dem Beispiel 5.

Beispiel 7
(Nacharbeitung des Beispiels 4 der US-PS 2 459 002)

Der Versuch wurde in einem 1 Mol Ansatz wie im Beispiel 4 der US-PS beschrieben als Eintopfverfahren ohne Zwischenisolierung des 3-Nitro-4-acetylaminotoluols mit anschließender saurer Spaltung durchgeführt.

Das Feuchtprodukt wurde bis zur Gewichtskonstanz im Vakuum bei 60°C getrocknet.
Ausbeute: Produkt trocken 89,0 g (50,5 % der Theorie); Reinheit: 86,4 %.

Beispiel 8 (Vergleichsbeispiel)

Es wird wie im Beispiel 7 verfahren, jedoch wird die heiße, wäßrige und neutrale Suspension nach der Wasserdampfdestillation mit 60 g (1,5 Mol) NaOH versetzt, auf 95°C erhitzt und 1 1/2 Stunden bei dieser Temperatur gehalten, wobei die Acetylaminogruppe zur Aminogruppe hydrolysiert. Es wird auf 60°C abgekühlt, 1/2 Stunde bei 60°C nachgerührt, abgesaugt und mit Wasser alkalifrei gewaschen. Das Feuchtprodukt wird bis zur Gewichtskonstanz im Vakuum bei 60°C getrocknet. Ausbeute: Produkt trocken 134,5 g (82,4 % der Theorie); Reinheit: 93,2 %.

Patentansprüche

1) Verfahren zur Herstellung von 3-Nitro-4-aminotoluol durch Nitrieren von 4-Tolylcarbaminsäureestern und anschließender alkalischer Verseifung und Aufarbeitung, dadurch gekennzeichnet, daß man die Nitrierung in Gegenwart von inerten, mit Wasser nicht mischbaren organischen Lösungs- und/oder Verdünnungsmitteln mit 50 bis 100 gew.-%iger Salpetersäure bei Temperaturen von -10 bis +45°C durchführt.

2) Verfahren nach Anspruch 1, dadurch gekennzeichnet, daß man als 4-Tolylcarbaminsäureester solche der allgemeinen Formel

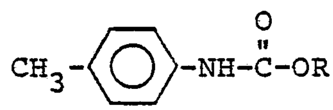 ,

worin

R für einen cycloaliphatischen oder aliphatischen Rest mit 1 bis 8 Kohlenstoffatomen steht,

einsetzt.

3) Verfahren nach Anspruch 2, dadurch gekennzeichnet, daß man den Methyl-, Ethyl-, n-Propyl-, iso-Propyl-, n-Butyl-, iso-Butyl-, tert.-Butyl-, n-Pentyl-, iso-Pentyl-, n-Hexyl- oder den Cyclohexylester der 4-Tolylcarbaminsäure einsetzt.

4) Verfahren nach Ansprüchen 1 bis 3, dadurch gekennzeichnet, daß man den 4-Tolylcarbaminsäureester und die Salpetersäure gleichzeitig dem vorgelegten

inerten organischen Lösungs- und/oder Verdünnungsmittel zugibt.

5) Verfahren nach Ansprüchen 1 bis 3, dadurch gekennzeichnet, daß man den 4-Tolylcarbaminsäureester und die Salpetersäure gleichzeitig einem vorgelegten Gemisch aus inerten organischen Lösungs- und/oder Verdünnungsmitteln und Salpetersäure zugibt.

6) Verfahren nach Ansprüchen 1 bis 5, dadurch gekennzeichnet, daß man pro Mol 4-Tolylcarbaminsäureester 1,5 bis 5 Mol 100 gew.-%ige Salpetersäure einsetzt.

7) Verfahren nach Ansprüchen 1 bis 5, dadurch gekennzeichnet, daß man pro Mol 4-Tolylcarbaminsäureester 3 bis 10 Mol 50 gew.-%ige Salpetersäure einsetzt.

8) Verfahren nach Ansprüchen 1 bis 7, dadurch gekennzeichnet, daß man pro Mol 4-Tolylcarbaminsäureester 400 bis 2000 ml inertes organisches Lösungs- und/oder Verdünnungsmittel einsetzt.

9) Verfahren nach Ansprüchen 1 bis 8, dadurch gekennzeichnet, daß man die Umsetzung bei Temperaturen von 0 bis 40°C durchführt.

10) Verfahren nach Ansprüchen 1 bis 9, dadurch gekennzeichnet, daß man die Umsetzung bei Temperaturen von 10 bis 25°C durchführt.

Europäisches Patentamt

# EUROPÄISCHER RECHERCHENBERICHT

0047337
Nummer der Anmeldung
EP 80106382.7

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.) |
|---|---|---|---|
| E | EP - A1 - 0 027 658 (BAYER AG)<br>* Patentanspruch 1 *<br>--<br>DE - C - 767 072 (G. SIEGLE & CO. GMBH)<br>* Gesamt *<br>---- | 1<br><br><br>1 | C 07 C 87/60 |

RECHERCHIERTE SACHGEBIETE (Int. Cl.)

C 07 C 87/00
C 07 C 79/00
C 07 C 125/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 09-11-1981 | HEIN |

EPA form 1503.1 06.78